# FASCICULE DE BREVET EUROPEEN

(11) **EP 1 845 935 B1**
(45) Date de publication et mention de la délivrance du brevet: **19.11.2008**
(21) Numéro de dépôt: 06709302.1
(22) Date de dépôt: 13.02.2006
(51) Int. Cl.: A61K 8/49, A61Q 5/00, A61Q 17/04, A61Q 19/04, A61K 31/353

(54) **UTILISATION DE LA SILYMARINE ET/OU DE SES CONSTITUANTS COMME AGENTS PROMOTEUR DE LA PIGMENTATION DE LA PEAU OU DES CHEVEUX**
VERWENDUNG VON SILYMARIN UND/ODER DEN BESTANDTEILEN DAVON ALS PROMOTOREN DER PIGMENTIERUNG VON HAUT ODER HAAREN
USE OF SILYMARINE AND/OR THE CONSTITUENTS THEREOF AS PROMOTERS OF THE PIGMENTATION OF THE SKIN OR HAIR

(30) Priorité: 11.02.2005 FR 0501446
(43) Date de publication de la demande: 24.10.2007
(73) Titulaire: Greenpharma, 63360 Saint Beauzire (FR); Bioalternatives SAS, 86160 Gencay (FR)
(72) Inventeur: BERNARD, Philippe, F-45000 Orléans (FR); BERNARD, François-Xavier, F-86160 Saint Maurice la Clouere (FR)
(74) Mandataire: Maureau, Philippe
(86) Numéro de dépôt international: PCT/FR2006/000320
(87) Numéro de publication internationale: WO 2006/085013

(56) Documents cités:
- EP-A- 0 680 744
- WO-A-20/04014413
- DE-A1- 4 323 614
- US-A1- 2002 155 074
- US-A1- 2005 002 962
- US-B1- 6 399 046
- PATENT ABSTRACTS OF JAPAN vol. 2003, no. 12, 5 décembre 2003 (2003-12-05) & JP 2004 002264 A (TOKAI UNIV), 8 janvier 2004 (2004-01-08)
- DATABASE WPI Section Ch, Week 200372 Derwent Publications Ltd., London, GB; Class B04, AN 2003-758887 XP002399123 & JP 2002 220333 A (KANEBO LTD) 9 août 2002 (2002-08-09)

## Description

La couleur de la peau humaine est fonction de différents facteurs et notamment de la race et du sexe, mais aussi de facteurs environnementaux (saison, exposition solaire...) ; elle est principalement dépendante de la nature et la concentration de mélanine produite par les mélanocytes. Les mélanocytes sont les cellules spécialisées qui, par l'intermédiaire d'organelles particuliers, les mélanosomes, synthétisent la mélanine. Certaines personnes présentent naturellement ou accidentellement des défauts plus ou moins localisés de pigmentation, nécessitant des traitements locaux palliatifs, ou sont demandeurs de traitements plus généraux, permettant de stimuler une pigmentation naturelle.

La pigmentation est naturellement une protection efficace contre les effets délétères des rayonnements ultraviolets et contre le photovieillissement cutané en général. La pigmentation cutanée est aussi une protection contre l'apparition de cancers de la peau ; pour des expositions solaires du même ordre, les personnes et ethnies à la peau sombre développent beaucoup moins de cancers cutanés que les personnes de peau pâle.

De la même manière, la couleur des poils et des cheveux est due à la mélanine. A différentes périodes de leur vie, notamment au cours du vieillissement, certaines personnes montrent une dépigmentation progressive de la chevelure, avec une diminution voire l'arrêt des processus de mélanogénèse dans les mélanocytes associés au bulbe pilaire. Il est très intéressant de pouvoir proposer des traitements préventifs ou curatifs susceptibles de maintenir le processus de pigmentation du cheveu ou de stimuler la mélanogénèse et la pigmentation des cheveux ayant tendance au blanchissement.

L'exposition solaire et les rayonnements UV ont des effets délétères sur les cheveux, à la fois au niveau de la tige pilaire (oxydation et décoloration), mais aussi, et de façon plus dommageable, sur le bulbe du follicule, pouvant conduire à la chute du cheveu. La récupération ou la stimulation d'une pigmentation du follicule est susceptible de limiter la chute du cheveu ou de stimuler sa repousse.

L'utilisation de substances pro-pigmentantes inoffensives, en application topique ou systémique dans des compositions, et présentant une bonne efficacité est tout particulièrement recherchée en vue de traiter les hypopigmentations régionales naturelles (d'origine génétique, leucodermies comme le vitiligo, vieillissement) ou accidentelles (cicatrices post-lésionnelle, mycoses fongiques), ainsi que la perte de pigmentation du cheveu liée au stress ou au cours du vieillissement.

L'utilisation de substances inoffensives, en application topique ou systémique dans des compositions, et protégeant le cheveu, limitant sa chute ou/et stimulant sa repousse, dans des conditions normales, de stress, d'exposition solaire, ou/et au cours du vieillissement est aussi d'intérêt majeur.

Le mécanisme de formation de la pigmentation de la peau est complexe et fait intervenir schématiquement les principales étapes suivantes :
Tyrosine -> Dopa -> Dopaquinone -> Dopachrome-> Mélanine. La mélanine est stockée dans des organites ou mélanosomes, puis transférée aux kératinocytes voisins.

Chacune de ces étapes est indispensable à la pigmentation. La tyrosinase (monophénol dihydroxyl phénylalanine : oxygen oxydo-reductase EC 1.14.18.1) est la première enzyme intervenant dans cette suite de réactions. Elle catalyse notamment la réaction de transformation de la tyrosine en Dopa (dihydroxyphénylalanine) grâce à son activité hydroxylase et la réaction de transformation de la Dopa en dopaquinone grâce à son activité oxydase. Cette tyrosinase n'agit que lorsqu'elle est à l'état de maturation sous l'action de certains facteurs biologiques ; une signalisation via des récepteurs spécifiques tels que les récepteurs à la mélanocortine (MCR) est mise en jeu pour l'induction du processus de synthèse de mélanine par les mélanocytes, notamment le récepteur MC1R.

Dans l'épiderme, le mélanocyte est impliqué dans l'unité mélanique épidermique qui comporte un mélanocyte entouré d'environ 36 kératinocytes voisins. Tous les individus, sans distinction de phototype, ont approximativement le même nombre de mélanocytes pour une zone cutanée donnée. Les différences ethniques, en terme de pigmentation, ne sont pas dues au nombre de mélanocytes, mais aux propriétés de leurs mélanosomes. Les mélanosomes sont agrégés en complexes et sont de petites tailles. Ce sont des organelles hautement spécialisées dont l'unique fonction est la production de mélanine. Au fur et à mesure que la mélanine est synthétisée dans les mélanosomes, ceux-ci se déplacent de la région périnucléaire vers l'extrémité des dendrites des mélanocytes. Par phagocytose, l'extrémité des dendrites est capturée par les kératinocytes, et les mélanosomes sont redistribués dans les kératinocytes. Les prolongements dendritiques des mélanocytes, ainsi que l'activité phagocytique des kératinocytes jouent donc un rôle essentiel dans le transfert de la mélanine. Le transfert de mélanosome est un phénomène phagocytique considéré comme classique, il met en jeu des récepteurs connus comme le « protease-activated receptor 2 » (PAR-2).

Bien que le taux de mélanine varie d'une population à une autre, la quantité de tyrosinase ne varie pas significativement et le taux d'ARN messager de la tyrosinase est identique dans des peaux blanches ou noires. Les variations dans la mélanogénèse sont donc dues à des variations soit de l'activité de la tyrosinase, soit de la capacité des kératinocytes à phagocyter les mélanosomes. Ceci indique que le kératinocyte est un acteur majeur de la pigmentation ; 1) c'est quantitativement le représentant majeur de l'unité mélanique, c'est aussi lui qui va influencer, via des molécules informationnelles (cytokines, hormones) un grande partie de l'activité mélanogénique ; 2) c'est sa capacité de phagocytose, associée à une présentation adéquate des mélanosomes, dans un réseau dendritique dense, qui va permettre la distribution optimale de la mélanine dans l'épiderme et la pigmentation.

Une substance est reconnue comme pro-pigmentante si elle agit directement ou indirectement sur l'activation du processus de synthèse de mélanine, et/ou si elle stimule la capacité de phagocytose des mélanosomes par les kératinocytes.

Les substances comme la alpha-melanotropin (alpha-melanocyte-stimulating hormone, alpha-MSH) et la corticotropin (adrenocorticotropic hormone, ACTH) stimulent la prolifération et la synthèse de mélanine par les mélanocytes, via la liaison à des récepteurs spécifiques, notamment le récepteur MC1-R.

Peu d'inducteurs naturels sont actuellement disponibles et utilisés pour la pigmentation naturelle mélanique de la peau ou des cheveux.

Il subsiste le besoin d'un nouvel agent promoteur de la pigmentation et/ou pigmentant de la peau humaine, des poils et/ou des cheveux à action plus efficace que ceux connus, et possédant une action renforcée de façon à pouvoir être utilisé en quantité faible sans effet secondaire au niveau de la peau.

Le chardon Marie (*Silybum marianum (L.)* Gaertner de la famille des *Asteraceae,* est une plante méditerranéenne, poussant également en Amérique du Nord et en Amérique du Sud, ainsi qu'en Australie, utilisée dans les pharmacopées traditionnelles, notamment contre diverses affections hépatiques.

La silymarine est un mélange de différents flavonolignanes issus de la taxifoline (un 2,3-dihydroflavanol) et de l'alcool coniférylique.

Ce mélange est majoritairement constitué de la silybine, (ou 2,3-Dihydro-3-(4-hydroxy-3-methoxyphenyl)-2-(hydroxymethyl)-6-(3,5,7-trihydroxy-4-oxobenzopyran-2-yl)benzodioxine), un benzodioxane trouvé sous deux formes de diastéréoisomères (7"R, 8"R et 7"S, 8"S) , la silybine et l'isosilybine ou leurs éniantiomères isolés.

Les autres constituants de la silymarine sont la silydianine, la silychristine, la silandrine, la silymonine et la taxifoline ou leurs énantiomères isolés.

La silymarine et/ou un de ses constituants purs ou en mélange possèdent des propriétés antioxydantes qui sont utilisées dans le traitement de diverses affections toxiques (notamment en tant qu'antihépatotoxique) et pour la favorisation de la régénération cellulaire.

On connaît de la demande de brevet WO9955326 l'utilisation de la silymarine dans la restauration du taux de glutathion dans les cellules, chez les mammifères

On connaît de EP 180505, l'utilisation de la silymarine dans des préparations cosmétiques destinées à retarder le vieillissement de la peau.

On connaît de WO 01/13879, l'utilisation cosmétique des huiles de silybum marianum, pour favoriser l'absorption cutanée des compositions comprenant en outre de la cynarine a effet radicalaire.

On connaît également de Carcinogenesis, vol 25, n°8, 1459-1465, 2004, l'utilisation de la Silybine pour la protection de la peau contre les dommages provoqués par les rayonnements UV.

La demande JP 2004 00264 divulgue quant à elle l'utilisation de la quercétine comme agent pigmentant de la peau.

On connait enfin de JP 2002 220333 et de US 2002/155074 l'utilisation de la sylimarine comme agent de dépigmentation de la peau.

Contre toute attente, les Demandeurs ont mis en évidence que la silybine et les autres constituants de silymarine et dérivés et/ou analogues de la silybine présentaient une bonne activité pro-pigmentante, même à faible concentration, sans faire preuve de cytotoxicité.

La présente invention a donc pour objet l'utilisation de la silymarine, ou de ses constituants principaux seul ou en mélange choisis parmi la silybine, (ou 2,3-Dihydro-3-(4-hydroxy-3-methoxyphenyl)-2-(hydroxymethyl)-6-(3,5,7-trihydroxy-4-oxobenzopyran-2-yl)benzodioxine), l'isosilybine, la silydianine, la silychristine, la silandrine et la silymonine, leurs éniantiomères isolés ainsi que leurs sels, pour la fabrication de compositions dermatologiques ou cosmétologiques destinées à induire, restaurer ou stimuler une pigmentation de la peau, des poils ou des cheveux.

L'utilisation des composés selon la présente invention permet d'induire, restaurer ou stimuler une pigmentation de la peau et des cheveux les protégeant ainsi des effets délétères de l'exposition solaire, incluant photovieillissement cutané, inflammation et érythèmes cutanés, chute de cheveux liée à l'exposition solaire, cancers cutanés et pathologies photo-induites.

Les constituants et/ou dérivés présent dans les compositions selon la présente invention présentent l'avantage d'agir sur plusieurs composantes majeures du mécanisme de la pigmentation, par exemple en modulant les activités MC1R et PAR-2 simultanément ou indépendamment l'une de l'autre.

Ils stimulent 1) la biosynthèse de la mélanine par les mélanocytes, 2) la formation d'un réseau dendritique dense chez le mélanocyte et 3) l'activité phagocytique des kératinocytes, augmentant ainsi à la fois la quantité de mélanine produite et l'efficacité du transfert des mélanosomes aux kératinocytes voisins.

Par ailleurs, s'agissant des cheveux, il a été mis en évidence, que 4) l'invention stimulait nettement la pigmentation du follicule (bulbe de cheveu).

Dans le cadre de la présente invention, des sels pharmaceutiquement et/ou cosmétiquement acceptables des composés cités, peuvent être utilisés. Parmi les sels on citera à titre d'exemple, les sels de sodium, les pentaacétates, les tribromures.

Des dérivés hémisynthétiques ou synthétiques peuvent également être utilisés.

Des dérivés glycosilés, des esters ou des éthers naturels ou synthétiques peuvent également être utilisés. On citera par exemple la sylimarine-n-methylglycamate, des esters avec l'acide hemisuccinique ou la silymarine pentaméthyl ou triméthyl éther.

Tous ces composés sont répertoriés dans les Chemical Abstracts, on citera par exemple la silymarine indexée sous les « Registry Numbers » 39468-33-2, 65666-07-1, 144160-532, 104444-08-8, 104444-07-7, sel de sodium sous le RN 66-580-75-4, N-methyl glycamate sous le RN 53026-30-5, hemisuccinate de sodium sous le RN 52691-96-0, pentaacétate, 27900-74-9, tribromure 27359-07-5, pentaméthyl éther 27359-05-3triméthyl éther 27359-04-2.

La silybine 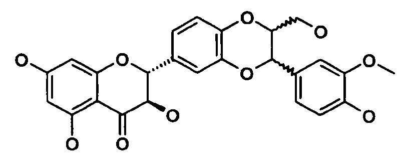 Silybine ou , 2-[(2R,3R)-2,3-dihydro-3-(4-hydroxy-3- methoxyphenyl)-2-(hydroxymethyl)-1,4-benzodioxin-6-yl]-2,3-dihydro-3,5,7-trihydroxy-4H-1-Benzopyran-4-one, (2R,3R)- (9CI) est indexée sous le RN 25888-70-6.

L'isosilybine ou, 2-[2,3-dihydro-2-(4-hydroxy-3-methoxyphenyl)-3-(hydroxymethyl)-1,4-benzodioxin-6-yl]-2,3-dihydro-3,5,7-trihydroxy-4H-1-Benzopyran-4-one,(2R,3R)-(9CI), est indexée sous le RN 72581-71-6.

La silydianine ou, 4-[(2R,3R)-3,4-dihydro-3,5,7-trihydroxy-4-oxo-2H-1-benzopyran-2-yl]-2,3,3a,7a-tetrahydro-7a-hydroxy-8-(4-hydroxy-3-methoxyphenyl)-3,6-Methanobenzofuran-7(6H)-one, (3R,3aR,6R,7aR,8R)- (9CI) est indexée sous le RN 29782-68-1.

La silychristine ou, 2-[(2R,3S)-2,3-dihydro-7-hydroxy-2-(4-hydroxy-3-methoxyphenyl)-3-(hydroxymethyl)-5-benzofuranyl]-2,3-dihydro-3,5,7-trihydroxy-4H-1-Benzopyran-4-one, (2R,3R)- (9CI) est indexée sous le RN 33889-69-9.

La silandrine ou, 2-[(2R,3R)-2,3-dihydro-2-(4-hydroxy-3-methoxyphenyl)-3-(hydroxymethyl)-1,4-benzodioxin-6-yl]-2,3-dihydro-5,7-dihydroxy-4H-1-Benzopyran-4-one, (2S)- (9CI) est indexée sous le RN 70815-2-6.

La silymonine ou , 4-[(2S)-3,4-dihydro-5,7-dihydroxy-4-oxo-2H-1-benzopyran-2-yl]-2,3,3a,7a-tetrahydro-7a-hydroxy-8-(4-hydroxy-3-methoxyphenyl)-3,6-Methanobenzofuran-7(6H)-one, (3R,3aR,6R,7aR,8R)- (9CI) est indexée sous le RN 70815-31-5.

La plupart des constituants de la sylimarine, existent sous forme d'isomères diastéréoisomères et/ou enantiomères.

Ces isomères peuvent être séparés par des techniques connues de l'homme de l'art, pour être utilisé sous forme optiquement pure comme agent actif dans des compositions selon l'invention.

On citera à ce titre la publication de David Y.W. Lee, J. Nat.Prod. 2003, 66, 1171-1174, qui décrit la séparation des isomères de la silybine et de l'isosylibine en silybine A, silybine B, isosilybine A et isosilybine B.

La silymarine est obtenue classiquement par extraction, notamment, de chardon Marie, les composés ci-dessus cités sont les principaux composés responsables de l'action thérapeutique de la plante.

Un composé convenant particulièrement bien à la mise en oeuvre de la présente invention est la 2,3-Dihydro-3-(4-hydroxy-3-methoxyphenyl)-2-(hydroxymethyl)-6-(3,5,7-trihydroxy-4-oxobenzopyran-2-yl)benzodioxine ou silybine, qui peut toutefois être utilisé en mélange dans le complexe dénommé silymarine ou bien purifié voire même énantiomériquement pure (utilisation d'un seul isomère).

Ce composé présente l'avantage d'être déjà utilisé en thérapeutique comme agent hépatoprotecteur. De nombreuses données concernant son innocuité son déjà connues et à disposition. De plus, son industrialisation est également déjà opérationnelle et peu coûteuse.

Dans un mode de réalisation le constituant de la silymarine est la silybine ou l'isosilybine énantiomériquement pure.

Dans ce mode de réalisation les constituants sont choisis parmi la silybine A, la silybineB, l'isosilybine A ou l'isosilybine B ou leurs sels seuls ou en mélange.

Selon un mode de réalisation, la sylimarine ou un de ses constituants seul ou en mélange sont obtenus par extraction d'une plante du genre *Sylibum* (notamment *Silybum marianum* (L.) Gaertn), de la famille des *Asteraceae.*

Lorsque l'agent actif est obtenu par extraction, on entend tout particulièrement l'obtention d'un extrait de *Silybum.* Il s'agit plus spécialement d'un extrait de cellules de *Silybum* et tout spécifiquement d'un extrait de cellules d'au moins un végétal du genre *Silybum* de la famille des Astéracées. Ce matériel cellulaire peut être obtenu par culture *in vitro* ou *in vivo.* Par culture *in vitro*, on entend l'ensemble des techniques connues de l'homme du métier qui permettent de manière artificielle l'obtention d'un végétal ou d'une partie d'un végétal. Par culture *in vivo,* on entend l'ensemble des techniques de cultures qui permettent d'obtenir un végétal ou une partie d'un végétal. Ainsi, l'extrait peut être un extrait d'organe (racine, tige, feuille, écorce), voire de cellules d'organe, d'au moins une plante du genre *Silybum* de la famille des Astéracées, ou encore un extrait de cellules indifférenciées d'au moins une telle plante. Ces extraits sont enrichis dans des proportions variables selon le type d'extrait, en flavonolignanes. Ces extraits purifiés présentent ainsi l'avantage d'être affranchis de tout problème de toxicité par rapport à l'extrait brut. Plus particulièrement, trois formules d'extraction/purification peuvent être envisagées : (i) un extrait total de la plante, (ii) un extrait visant à concentrer les flavonolignanes dérivées de la taxifoline et enfin, (iii) l'obtention de taxifoline et dérivés purs.

Toute méthode d'extraction ou de purification connue de l'homme du métier peut être utilisée selon l'invention. On peut en particulier citer les extraits alcooliques (notamment méthanoliques, éthanoliques), aqueux ou des extraits utilisant des solvants tels que les cétones, les esters, les éthers, les polyols, les solvants chlorés et les mélanges d'au moins deux des solvants précités, comme les extraits hydroalcooliques.

Selon un mode de réalisation, la sylimarine ou un de ses constituants seul ou en mélange sont obtenus par hémisynthèses ou synthèses.

L'invention concerne donc l'utilisation des composés décrits ci-dessus dans une composition, comme agent pigmentant de la peau et/ou des poils et/ou des cheveux. Il s'agira ainsi de compositions dermatologiques ou cosmétiques comprenant comme agent actif au moins un composé de structure générale (I) ou un dérivé ou analogue de celle-ci ou encore au moins un extrait végétal contenant au moins un composé de structure générale (I), ledit agent actif pouvant être associé avantageusement dans la composition avec un véhicule compatible et approprié au mode d'administration choisie.

Par exemple, pour une utilisation cosmétique et dermatologique, les compositions de l'invention peuvent se présenter sous la forme de crèmes, gels, lotions, laits, émulsions H/E et E/H, solutions, onguents, pulvérisateurs, huiles corporelles, lotions capillaires, shampooings, lotions après-rasages, savons, bâtons protecteur des lèvres, bâtons et crayons pour maquillage.

Il s'agira alors de compositions dermatologiques ou cosmétiques comprenant comme agent actif la silymarine, ou un de ses constituants principaux seul ou en mélange choisis parmi la silybine, (ou 2,3-Dihydro-3-(4-hydroxy-3-methoxyphenyl)-2-(hydroxymethyl)-6-(3,5,7-trihydroxy-4-oxobenzopyran-2-yl)benzodioxine),l'isosilybine, la silydianine, la silychristine, la silandrine et la silymonine ainsi que leurs sels, ledit agent actif pouvant être associé avantageusement dans la composition avec un véhicule compatible et approprié au mode d'administration choisie.Sous la forme de gel, elles comprennent des excipients appropriés tels que les esters de cellulose ou d'autres agents gélifiants, tels que le carbopol, la gomme guar.

Ces compositions cosmétiques et dermatologiques peuvent aussi prendre la forme de lotion ou solution dans laquelle les extraits et/ou molécules sont sous forme encapsulée, par exemple dans des microsphères. Ces microsphères peuvent par exemple être constituées de corps gras, d'agar et d'eau. Les agents actifs peuvent être aussi incorporés dans des vecteurs de type liposomes, glycosphères, cyclodextrines, dans des chylomicrons, des macro-, micro-, nano-particules ainsi que les macro-, micro- et nanocapsules et aussi être adsorbés sur des polymères organiques poudreux, les talcs, bentonites et autres supports minéraux. Ces émulsions jouissent d'une bonne stabilité et peuvent être conservées pendant le temps nécessaire pour l'utilisation à des températures comprises entre 0 et 50 °C sans qu'il y ait sédimentation des constituants ou séparation des phases.

Les compositions cosmétiques de l'invention comprennent de l'ordre de 0,01 à 10% en poids, préférentiellement entre 0,1 et 2,5% d'agents actifs lorsqu'elles sont sous forme de poudre et de l'ordre de 0,01 à 2,5%, préférentiellement entre 0,5 et 10% lorsqu'elles sont sous forme encapsulée.

Pour la préparation de ces compositions, la silymarine, ou un de ses constituants principaux seul ou en mélange choisis parmi la silybine, (ou 2,3-Dihydro-3-(4-hydroxy-3-methoxyphenyl)-2-(hydroxymethyl)-6-(3,5,7-trihydroxy-4-oxobenzopyran-2-yl)benzodioxine),l'isosilybine, la silydianine, la silychristine, la silandrine et la silymonine, ainsi que leurs sels ou un extrait végétal, sont mélangés aux excipients généralement employés en cosmétique.

Les compositions cosmétiques de l'invention peuvent aussi contenir des additifs ou des adjuvants usuels en cosmétologie, comme par exemple des agents antibactériens ou des parfums mais aussi des lipides d'extraction et/ou de synthèse, polymères gélifiants et viscosants, tensio-actifs et émulsifiants, principes actifs hydro ou liposolubles, extraits de plantes, extraits tissulaires, extraits marins, actifs de synthèse.

Les compositions cosmétiques de la présente invention peuvent aussi comprendre d'autres principes actifs complémentaires choisis pour leur action, par exemple pour la protection solaire, l'effet anti-rides, l'activité antiradicalaire et antioxydante, l'activité anti-irritante, la nutrition cellulaire, la respiration cellulaire, l'hydratation et la régénération cellulaire, les traitements anti-séborrhéïques, ainsi que d'autres principes actifs ayant une action sur la tonicité cutanée, la protection du cheveu.

Les compositions cosmétiques de la présente invention sont de préférence à utiliser quotidiennement en les appliquant une ou plusieurs fois par jour.

Les compositions cosmétiques de la présente invention sont très bien tolérées, elles ne présentent aucune phototoxicité et leur application sur la peau, pour des périodes de temps prolongées, n'implique aucun effet systémique.

Une application par voie orale peut également être envisagée. Il convient donc de s'intéresser également aux compositions comprenant au moins la silymarine, ou un de ses constituants principaux seul ou en mélange choisis parmi la silybine, (ou 2,3-Dihydro-3-(4-hydroxy-3-methoxyphenyl)-2-(hydroxymethyl)-6-(3,5,7-trihydroxy-4-oxobenzopyran-2-yl)benzodioxine), l'isosilybine, la silydianine, la silychristine, la silandrine et la silymonine, ainsi que leurs sels, associée à un véhicule ou à un excipient acceptable sur le plan pharmaceutique, cosmétique et dermatologique.

Les compositions dermocosmétiques se présentent sous forme liquide, de poudre, de pâte ou d'émulsion, seule ou en combinaison avec d'autres substances. Elles comprennent de l'ordre de 0,01 à 25% en poids de flavonolignanes, et plus particulièrement de silybine, de son précurseur biosynthétique la taxifoline et dérivés, de silychristine ou d'un extrait végétal les contenant.

Pour les préparations de ces compositions dermocosmétiques, les extraits et/ou composés purs ci-dessus cités, seuls ou en mélange et/ou sous forme de sels sont mélangés à des excipients.

Les exemples qui suivent illustrent l'invention sans en limiter la portée.

### Exemple 1 : Mise en évidence de l'activité sur la mélanogénèse dans des cultures de mélanocytes.

Un test biologique a mis en évidence l'activité stimulante de synthèse de mélanine par la silybine

L'effet stimulant de la mélanogénèse de la silybine a été mesuré sur cultures de mélanocytes humains normaux.

Pour la silybine, ont été déterminés :
- après 10 jours de culture dans des conditions standard, en plaques 24 puits, milieu Promocell sans « phorbol myristate acétate » (PMA) :
   la cytotoxicité, en estimant la réduction du « methyl thiazolyl tetrazolium » (MTT), la quantité de protéines, par dosage selon la méthode de Bradford et l'observation des tapis cellulaires,
   la quantité de mélanine présente dans les cultures, par mesure spectrophotométrique de la mélanine produite, après extraction alcaline, rapportée à 100 % du témoin (le témoin correspond au test réalisé sans composé à tester).
- après 3 jours de culture dans des conditions standard :
   les effets sur la prolifération des mélanocytes humains normaux par incorporation de thymidine tritiée dans l'ADN (marquage pendant les 24 dernières heures),
   les effets sur le métabolisme des mélanocytes humains normaux par incorporation de leucine tritiée dans les protéines néosynthétisées (marquage pendant les 24 dernières heures).

Les résultats sont rassemblés dans les tableaux suivants :
Synthèse de mélanine par les mélanocytes humains normaux :

| Traitement | Concentration | mélanine (µg/ml) | sd | n | % | p | protéines (mg/ml) | MTT (%) |
|---|---|---|---|---|---|---|---|---|
| Témoin | - | 27.7 | 0.38 | 3 | 100 | - | 0.645 | 100 |
| | 0.001% | 41.5 | 0.18 | 3 | 150 | P < 0.01 | 0.830 | 128 |
| Silybine | 0.0002% | 37.1 | 0.97 | 3 | 134 | P < 0.01 | 0.792 | 126 |
| | 0.00004% | 31.9 | 0.68 | 3 | 115 | P < 0.01 | 0.765 | 113 |

Dans tous les cas : concentrations en % (p/v) ; sd, écart-type ; p, significativité statistique.

Effets sur la prolifération et sur la néosynthèse de protéines de mélanocytes humains normaux:

| prolifération (3H-thymidine), mélanocytes humains normaux | | | | | | |
|---|---|---|---|---|---|---|
| Traitement | Concentration | cpm | sd | n | % | *p* |
| Témoin | - | 6805 | 0 | 3 | 100 | - |
| Silybine | 0.001% | 3622 | 750 | 6 | 53 | *P <* 0.01 |
| | 0.0002% | 5638 | 1104 | 6 | 83 | P *> 0.05* |

| synthèse protéique (3H-leucine), mélanocytes humains normaux | | | | | | |
|---|---|---|---|---|---|---|
| Traitement | Concentration | cpm | sd | n | % | *p* |
| Témoin | - | 9751 | 993 | 12 | 100 | - |
| Silybine | 0.001% | 12099 | 1995 | 6 | 124 | *P < 0.01* |
| | 0.0002% | 11705 | 486 | 6 | 120 | *P* < *0.05* |

| | | | | | | |
|---|---|---|---|---|---|---|
| cpm, coups par minute. | | | | | | |

La silybine a donc provoqué une augmentation significative de la production de mélanine dans les cultures de mélanocytes humains traités. Cette stimulation s'accompagne d'une augmentation modérée apparente du métabolisme des mélanocytes (MTT, protéines). Cet effet n'est pas du à une augmentation de la prolifération des mélanocytes (incorporation de thymidine), au contraire le produit a, dans ces conditions artificielles, une tendance à limiter la prolifération, sans aucune activité cytotoxique. D'autre part, la stimulation de l'activité métabolique sur des tests de plus courte durée (néosynthèse protéique, sur 72 h) confirme un très faible effet stimulant du métabolisme des mélanocytes (synthèse protéique augmentée de seulement 20 %).

Le produit n'a d'autre part pas montré d'effet sur la prolifération/synthèse protéique des kératinocytes.

L'effet stimulant de la mélanogénèse de la silybine a été mesuré sur cellules de mélanome murin de lignée B16F10.

Pour la silybine, ont été déterminés :
- après 7 jours de culture dans des conditions standard, en plaques 24 puits, milieu DMEM contenant 10 % de sérum de veau :
   la cytotoxicité, en estimant l'hydrolyse du MTT, la quantité de protéines, par dosage selon la méthode' de Bradford et l'observation des tapis cellulaires,
      la quantité de mélanine présente dans les cultures, par mesure spectrophotométrique de la mélanine produite, après extraction alcaline, rapportée à 100 % du témoin (le témoin correspond au test réalisé sans composé à tester).

Les résultats sont rassemblés dans le tableau suivant :
Synthèse de mélanine par les mélanocytes de lignée B16F10 :

| Traitement | Concentration | Mélanine (µg/ml) | sd | n | % | *p* | protéines (mg/ml) | *MTT (%)* |
|---|---|---|---|---|---|---|---|---|
| Témoin | - | 13.0 | 0.31 | 3 | 100 | - | *1.565* | *100* |
| Silybine | 0.001% | 33.0 | 0.83 | 3 | 254 | *P < 0.01* | *1.457* | *89* |
| | 0.0002% | 15.0 | 0.35 | 3 | 115 | *P < 0.01* | *1.594* | *100* |
| | 0.00004% | 13.0 | 0.58 | 3 | 100 | *P>0.05* | *1.553* | *100* |

La silybine a donc provoqué une augmentation très nette de la production de mélanine dans les cultures de mélanocytes de lignée B16F10 (250 % du témoin à 0.001 %), ce qui confirme l'effet pro-pigmentant observé, sur un second modèle cellulaire. Dans ce cas, la stimulation est observée sans modification du métabolisme cellulaire, selon les paramètres MTT et synthèse protéique, ce qui confirme la spécificité de la stimulation de la synthèse de mélanine.

La liaison (« binding ») de la silybine au récepteur MC1-R (« melanocortin 1 receptor ») a été étudiée. Des données structurales ont fait suspecter une possibilité de « binding » de ces composés au récepteur MC1-R (« melanocortin 1 receptor ») qui est fortement impliqué dans la mélanogénèse.

La silybine a été testé dans un test de déplacement d'un ligand MC1-R extrêmement puissant : déplacement de [¹²⁵I]NDP-alpha-MSH, (selon Siegrist et al, 1988, J. Recept. Res., 8 : 323-343).

Le ligand a une affinité de l'ordre de 10⁻¹⁰ M. La silybine aux concentrations de 100 µM (0.005 %) et supérieures déplace de façon significative ce ligand (10 % à 100 µM ; 30 % à 500 µM ; les concentrations supérieures n'ont pas été testées pour des raisons de solubilité), indiquant un impact spécifique sur le récepteur MC1-R. Notons également un effet de ces composés sur l'inhibition des phosphodiestérases, notamment de type 4: AMPc augmenté.

### Exemple 2 : Mise en évidence de l'activité sur la dendricité des mélanocytes :

Le but de ce test est de montrer l'effet des composés utilisés selon l'invention sur la modulation morphologique des mélanocytes.

Méthode: Les mélanocytes humains normaux sont traités dès l'ensemencement avec les composés selon l'invention pendant 2 jours, dans les conditions des essais de mélanogenèse sur mélanocytes humains, puis marqués par la CFDA (« carboxyfluorescein succinimidyl ester diacetate ») et observés en microscopie à fluorescence (coloration verte).

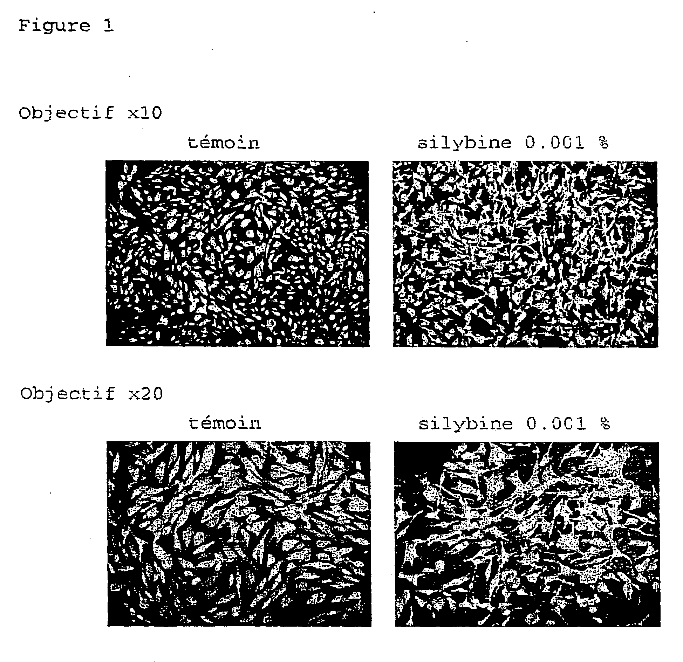

Observations ; selon figure 1.

Résultats: En absence des composés selon l'invention, les mélanocytes en culture sont peu dendritiques, voire bipolaires. En présence des composés selon l'invention, les mélanocytes montrent une dendricité nettement plus importante.

### Exemple 3 : Mise en évidence de l'activité sur la phagocytose des kératinocytes :

Le but de ce test est de montrer l'effet des composés utilisés selon l'invention sur la modulation de la phagocytose de particules par les kératinocytes.

Méthode: Des kératinocytes humains normaux sont cultivés en milieu KSFM et traités pendant 24 h avec les composés selon l'invention, en présence de billes fluorescentes calibrées (Molecular Probes) de la taille des mélanosomes. La phagocytose des particules est visualisée en microscopie à fluorescence, puis les cellules sont récoltées après trypsination et analysées par cytométrie de flux, pour le nombre de cellules ayant phagocyté un nombre seuil de particules et l'intensité de la fluorescence globale phagocytée (10 000 cellules analysées par condition, en triplicata)

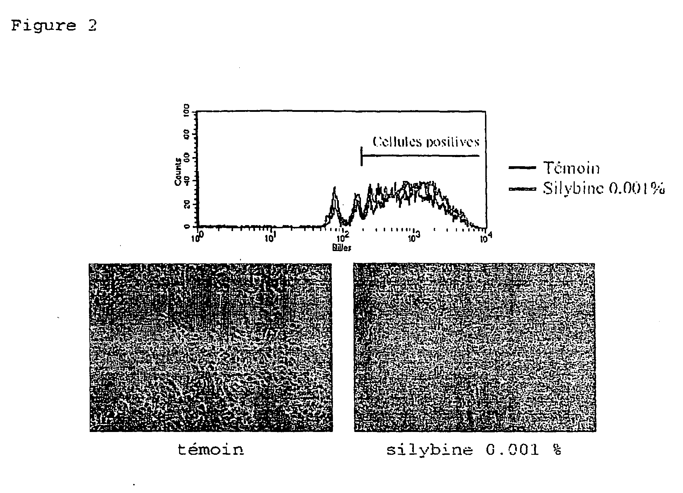

Observations: Les effets de la silybine sont rapportés dans le tableau suivant et la figure 2:

| Traitement | Concentration | % cellules positives* | Moyenne | SD | % Témoin | *p* |
|---|---|---|---|---|---|---|
| Témoin | | 35.39 | | | | |
| | - | 33.8 | 36 | 2 | 100 | |
| | | 38.02 | | | | - |
| Silybine | | 52.19 | | | | |
| | 0.001% | 50.74 | 51 | 1 | 143 | *p<0.01* |
| | | 49.94 | | | | |
| | | 45.88 | | | | |
| | 0.0002% | 44.29 | 44 | 1 | 124 | *p<0.01* |
| | | 42.92 | | | | |

| | | | | | | |
|---|---|---|---|---|---|---|
| * cellules ayant une phagocytose supérieure au double de la phagocytose basale | | | | | | |

Résultats: La silybine induit une stimulation significative et reproductible de la phagocytose de particules fluorescentes assimilables à des mélanosomes ; cette stimulation dose-dépendante a été mesurée par cytométrie de flux et par observation directe des tapis cellulaires en microscopie à fluorescence.

Exemple 4 : Mise en évidence de l'activité pro-pigmentante du cheveu : Le but de ce test est de montrer l'effet des composés utilisés selon l'invention sur synthèse de mélanine dans le follicule et sa pigmentation.

Méthode: Des follicules de cheveux humains normaux sont isolés par microdissection de scalp humain (lifting) et cultivés individuellement *in vitro,* en plaques 24 puits, selon Philpott et al, 1990, J. Cell. Sci., 3 : 463-471, avec un minimum de 15 cheveux de pigmentation homogène par condition. Les follicules sont cultivés pendant 7 jours en présence des composés selon l'invention. Les follicules sont photographiés à J0 et à J7 et la pigmentation est évaluée visuellement.

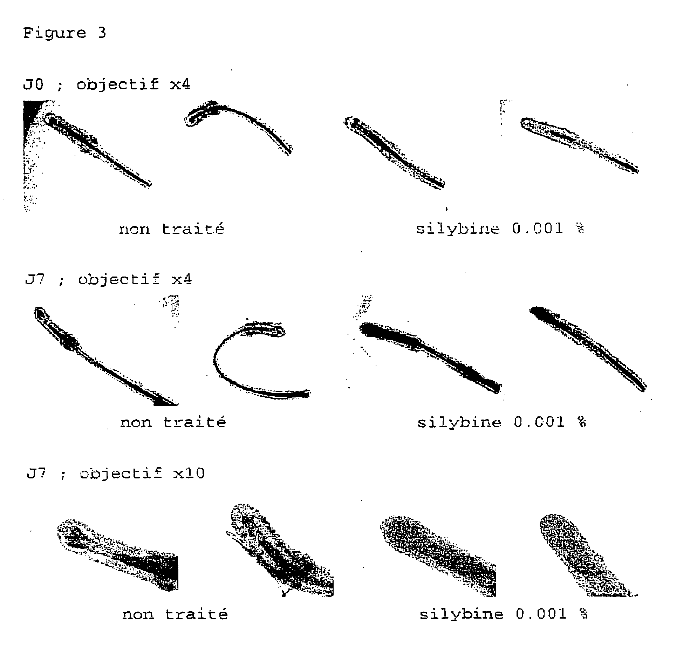

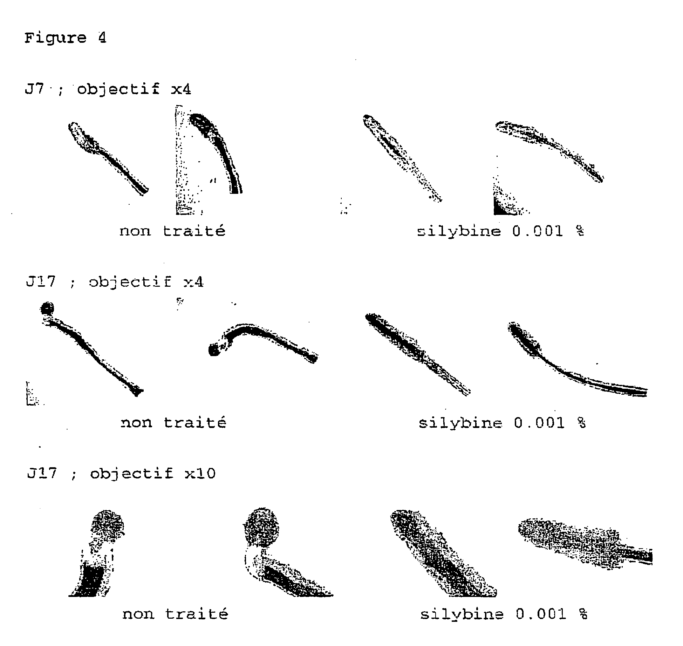

Observations: Les effets de la silybine sont rapportés dans la figure 3.

Résultats: La silybine n'est pas toxique pour le cheveu et permet un allongement de la tige pilaire, il induit une pigmentation très nette du bulbe et de la tige pilaire néoformée par rapport aux témoins non traités. Les composés utilisés selon l'invention stimulent donc la synthèse de mélanine dans le follicule et sa pigmentation.

## Revendications

1. Utilisation de la silymarine, ou de ses constituants principaux seul ou en mélange choisis parmi la silybine, (ou 2,3-Dihydro-3-(4-hydroxy-3-methoxyphenyl)-2-(hydroxymethyl)-6-(3,5,7-trihydroxy-4-oxobenzopyran-2-yl)benzodioxine), l'isosilybine, la silydianine, la silychristine, la silandrine et la silymonine, leurs énantiomères isolés ainsi que leurs sels, pour la fabrication de compositions dermatologiques destinées à induire, restaurer ou stimuler une pigmentation de la peau, des poils ou des cheveux.

2. Utilisation cosmétique de la silymarine, ou de ses constituants principaux seul ou en mélange choisis parmi la silybine, (ou 2,3-Dihydro-3-(4-hydroxy-3-methoxyphenyl)-2-(hydroxymethyl)-6-(3,5,7-trihydroxy-4-oxobenzopyran-2-yl)benzodioxine), l'isosilybine, la silydianine, la silychristine, la silandrine et la silymonine, leurs énantiomères isolés ainsi que leurs sels, comme agents destinés à induire, restaurer ou stimuler une pigmentation de la peau, des poils ou des cheveux.

3. Utilisation selon l'une quelconque des revendications précédentes **caractérisée en ce que** la sylimarine ou un de ses constituants seul ou en mélange sont obtenus par extraction d'une plante du genre Sylibum.

4. Utilisation selon l'une quelconque des revendications précédentes **caractérisée en ce que** la sylimarine ou un de ses constituants seul ou en mélange sont obtenus par hémisynthèses ou synthèses.

5. Utilisation selon l'une des revendications précédentes **caractérisée en ce que** le constituant de la silymarine est la 2,3-Dihydro-3-(4-hydroxy-3-methoxyphenyl)-2-(hydroxymethyl)-6-(3,5,7-trihydroxy-4-oxobenzopyran-2-yl)benzodioxine ou silybine.

6. Utilisation selon l'une des revendications 1 à 4 **caractérisée en ce que** le constituant de la silymarine est l'isosilybine.

7. Utilisation selon l'une des revendications 1 à 4 **caractérisée en ce que** le constituant de la silymarine est la silybine ou l'isosilybine énantiomériquement pure.

8. Utilisation selon quelconque des revendications précédentes **caractérisée en ce que** les compositions comprennent de l'ordre de 0,01 à 10% en poids d'agents actifs.

9. Utilisation selon quelconque des revendications précédentes **caractérisée en ce que** les compositions comprennent de l'ordre de 0,1 et 2,5% en poids d'agents actifs.

10. Utilisation selon quelconque des revendications précédentes **caractérisée en ce que** les compositions comprennent de l'ordre de 0,01 à 2,5% en poids d'agents actifs.

## Claims

1. Use of silymarin, or of the main constituents thereof alone or as a mixture, chosen from silybin (or 2,3-dihydro-3-(4-hydroxy-3-methoxyphenyl)-2-(hydroxymethyl)-6-(3,5,7-trihydroxy-4-oxobenzopyran-2-yl)benzodioxine), isosilybin, silydianin, silychristin, silandrin and silymonin, isolated enantiomers thereof and also salts thereof, for the manufacture of dermatological compositions for inducing, restoring or stimulating pigmentation of the skin, body hair or head hair.

2. Cosmetic use of silymarin, or of the main constituents thereof alone or as a mixture, chosen from silybin (or 2,3-dihydro-3-(4-hydroxy-3-methoxyphenyl)-2-(hydroxymethyl)-6-(3,5,7-trihydroxy-4-oxobenzopyran-2-yl)benzodioxine), isosilybin, silydianin, silychristin, silandrin and silymonin, isolated enantiomers thereof and also salts thereof, as agents for inducing, restoring or stimulating pigmentation of the skin, body hair or head hair.

3. Use according to either of the preceding claims, **characterized in that** silymarin or one of the constituents thereof alone or as a mixture is obtained by extraction of a plant of the genus *Silybum.*

4. Use according to any one of the preceding claims, **characterized in that** silymarin or one of the constituents thereof alone or as a mixture is obtained by semi-synthesis or synthesis.

5. Use according to one of the preceding claims, **characterized in that** the silymarin constituent is 2,3-dihydro-3-(4-hydroxy-3-methoxyphenyl)-2-(hydroxymethyl)-6-(3,5,7-trihydroxy-4-oxobenzopyran-2-yl)benzodioxine or silybin.

6. Use according to one of Claims 1 to 4, **characterized in that** the silymarin constituent is isosilybin.

7. Use according to one of Claims 1 to 4, **characterized in that** the silymarin constituent is enantiomerically pure silybin or isosilybin.

8. Use according to any one of the preceding claims, **characterized in that** the compositions comprise from about 0.01% to 10% by weight of active agents.

9. Use according to any one of the preceding claims, **characterized in that** the compositions comprise from about 0.1% to 2.5% by weight of active agents.

10. Use according to any one of the preceding claims, **characterized in that** the compositions comprise from about 0.01% to 2.5% by weight of active agents.

## Patentansprüche

1. Verwendung von Silymarin oder seinen Hauptbestandteilen, allein oder als Mischung, ausgewählt aus der Gruppe Silybin (bzw. 2,3-Dihydro-3-(4-hydroxy-3-methoxyphenyl)-2-(hydroxymethyl)-6-(3,5,7-trihydroxy-4-oxobenzopyran-2-yl)benzodioxin), Isosilybin, Silydianin, Silychristin, Silandrin und Silymonin, ihren isolierten Enantiomeren sowie ihren Salzen für die Herstellung von dermatologischen Zusammensetzungen zur Induktion, Wiederherstellung oder Stimulation einer Pigmentierung der Haut, des Körperhaars oder des Kopfhaares.

2. Kosmetische Verwendung von Silymarin oder seinen Hauptbestandteilen, allein oder als Mischung, ausgewählt aus der Gruppe Silylbin (bzw. 2,3-Dihydro-3-(4-hydroxy-3-methoxyphenyl)-2-(hydroxymethyl-6-(3,5,7-trihydroxy-4-oxobenzopyran-2-yl)benzodioxin), Isosilybin, Silydianin, Silychristin, Silandrin, und Silymonin, ihren isolierten Enantiomeren sowie ihren Salzen als Mittel zur Induktion, Wiederherstellung oder Stimulation einer Pigmentierung der Haut, des Körperhaars oder des Kopfhaares.

3. Verwendung nach einem der vorhergehenden Ansprüche, **dadurch gekennzeichnet, daß** das Silymarin oder eines seiner Bestandteile, allein oder als Mischung, durch Extrahieren einer Pflanze der Gattung Silybum erhalten werden.

4. Verwendung nach einem der vorhergehenden Ansprüche, **dadurch gekennzeichnet, daß** das Silymarin oder eines seiner Bestandteile, allein oder als Mischung, durch Halbsynthesen oder Synthesen erhalten wird.

5. Verwendung nach einem der vorhergehenden Ansprüche, **dadurch gekennzeichnet, daß** es sich bei dem Silymarinbestandteil um 2,3-Dihydro-3-(4-hydroxy-3-methoxyphenyl)-2-(hydroxymethyl)-6-(3,5,7-trihydroxy-4-oxobenzopyran-2-yl)benzodioxin bzw. Silybin handelt.

6. Verwendung nach einem der Ansprüche 1 bis 4, **dadurch gekennzeichnet, daß** es sich bei dem Silymarinbestandteil um Isosilybin handelt.

7. Verwendung nach einem der Ansprüche 1 bis 4, **dadurch gekennzeichnet, daß** es sich bei dem Silymarinbestandteil um Silybin oder um enantiomerenreines Isosilybin handelt.

8. Verwendung nach einem der vorhergehenden Ansprüche, **dadurch gekennzeichnet, daß** die Zusammensetzungen im Bereich von 0,01 bis 10 Gew.-% Wirkstoffe enthalten.

9. Verwendung nach einem der vorhergehenden Ansprüche, **dadurch gekennzeichnet, daß** die Zusammensetzungen im Bereich von 0,1 bis 2,5 Gew.-% Wirkstoffe enthalten.

10. Verwendung nach einem der vorhergehenden Ansprüche, **dadurch gekennzeichnet, daß** die Zusammensetzungen im Bereich von 0,01 bis 2,5 Gew.-% Wirkstoffe enthalten.
